# EUROPEAN PATENT APPLICATION

(11) **EP 3 326 520 A1**
(43) Date of publication of application: **30.05.2018**
(21) Application number: 17203123.9
(22) Date of filing: 22.11.2017
(51) Int. Cl.: A61B 5/00

(54) **PHOTOACOUSTIC APPARATUS, INFORMATION PROCESSING METHOD**

(30) Priority: 25.11.2016 JP 2016229315; 09.08.2017 JP 2017154457
(71) Applicant: CANON KABUSHIKI KAISHA, Ohta-ku Tokyo 146-8501 (JP)
(72) Inventor: ABE, Naoto, Ohta-ku, Tokyo 146-8501 (JP)
(74) Representative: Walker, Philip Martin

(57) **Abstract**

The present disclosure is directed to providing an apparatus and a method for suitably displaying a photoacoustic image for assisting diagnosis using an ultrasound image when restricting the degradation of diagnostic capability. An information processing method according to the present disclosure includes displaying an ultrasound image through transmission and reception of an ultrasonic wave to/from a subject, setting a partial region of the ultrasound image as a region of interest when the ultrasound image is being displayed, setting a light irradiation condition including a light quantity and a repetition frequency of irradiation light to the subject according to the region of interest, and receiving a photoacoustic wave generated through light irradiation to the subject under the light irradiation condition, and displaying a photoacoustic image of a region corresponding to the region of interest.

## Description

### BACKGROUND OF THE INVENTION

### Field of the Invention

The present invention relates to an apparatus and a method for displaying a photoacoustic image based on a photoacoustic wave generated by a photoacoustic effect.

### Description of the Related Art

As an image diagnosis apparatus for non-invasively imaging a state inside a living body, an ultrasonic diagnosis apparatus is known to generate an ultrasound image through the transmission and reception of an ultrasonic wave. The ultrasonic diagnosis apparatus generates an ultrasound image based on a received signal, i.e., a transmitted wave or reflected wave (ultrasonic echo) of the transmitted ultrasonic wave.

On the other hand, as an image diagnosis apparatus for non-invasively imaging a status inside a living body, a photoacoustic apparatus is known to use an ultrasonic wave (photoacoustic wave) generated when a biological tissue irradiated with light adiabatically expands by the light emission energy. The photoacoustic apparatus generates a photoacoustic image based on a received signal of a photoacoustic wave.

Japanese Patent Application Laid-Open No. 2012-196430 discusses a switch for switching between an operation mode for detecting a reflected ultrasound wave and an operation mode for detecting a photoacoustic wave. Japanese Patent Application Laid-Open No. 2012-196430 also discusses a technique for switching between a display of an ultrasound image and a superimposed display of the ultrasound image and a photoacoustic image by using the switch.

### SUMMARY OF THE INVENTION

It is assumed that, in diagnosis using an ultrasound image and a photoacoustic image, the ultrasound image is used as a basic diagnostic image like a conventional ultrasonic diagnosis apparatus, and the photoacoustic image is displayed as an image playing a supplementary role in diagnosis based on the ultrasound image. However, switching from the display of the ultrasound image to the superimposed display of the ultrasound and photoacoustic images may degrade diagnostic capability.

The present invention is directed to providing an apparatus and a method for suitably displaying a photoacoustic image for assisting diagnosis by an ultrasound image while suppressing the degradation of diagnostic capability.

According to a first aspect of the present invention, there is provided an information processing method as specified in claims 1 to 9. According to a second aspect of the present invention, there is provided a program as specified in claim 10. According to a third aspect of the present invention, there is provided a photoacoustic apparatus as specified in claim 11. According to another aspect there is provided an information processing apparatus according to claim 16.

Further features of the present invention will become apparent from the following description of embodiments with reference to the attached drawings.

### BRIEF DESCRIPTION OF THE DRAWINGS

Fig. 1 is a schematic view illustrating a region of interest set on an ultrasound image.
Fig. 2 is a block diagram illustrating a photoacoustic apparatus according to an embodiment.
Fig. 3 is a schematic view illustrating a handheld probe according to the embodiment.
Fig. 4 is a block diagram illustrating a computer and a peripheral configuration according to the embodiment.
Fig. 5 is a flowchart illustrating an image display method according to the embodiment.
Figs. 6A and 6B are timing charts according to the embodiment.
Fig. 7 is a schematic view illustrating another example of a region of interest set on an ultrasound image.
Fig. 8 is a block diagram illustrating storage data according to the embodiment.
Figs. 9A, 9B, and 9C are other timing charts according to the embodiment.

### DESCRIPTION OF THE EMBODIMENTS

The present invention relates to an apparatus for acquiring information related to an irradiation target through light irradiation. More specifically, the present invention relates to an apparatus for acquiring photoacoustic image data originated from a photoacoustic wave generated through light irradiation. The acoustic wave generated by a photoacoustic effect according to the present invention is typically an ultrasonic wave, and includes a sound wave, an acoustic wave, and a photoacoustic wave.

The photoacoustic image data according to the present invention conceptually includes all of image data originated from a photoacoustic wave generated through light irradiation. For example, the photoacoustic image data represents the spatial distribution of at least one piece of subject information including the photoacoustic wave generation sound pressure (initial sound pressure), the optical absorption energy density, the optical absorption coefficient, and the density (such as the oxygen saturation) of a constituent of the subject. Subject information acquired based on the photoacoustic wave generated through light irradiation with a plurality of different wavelengths is spectrum information such as the density of a constituent of the subject. The spectrum information may be the oxygen saturation, the oxygen saturation weighted with an intensity such as the absorption index, total hemoglobin concentration, oxyhemoglobin concentration, or deoxyhemoglobin concentration. The spectrum information may be the glucose concentration, collagen concentration, melanin concentration, or volume fraction of fat or water.

Ultrasound image data obtained by the apparatus according to the present embodiment includes at least one piece of image data such as the B mode image, Doppler image, and elastography image. Ultrasound images conceptually include all of images obtained through transmission and reception of an ultrasonic wave.

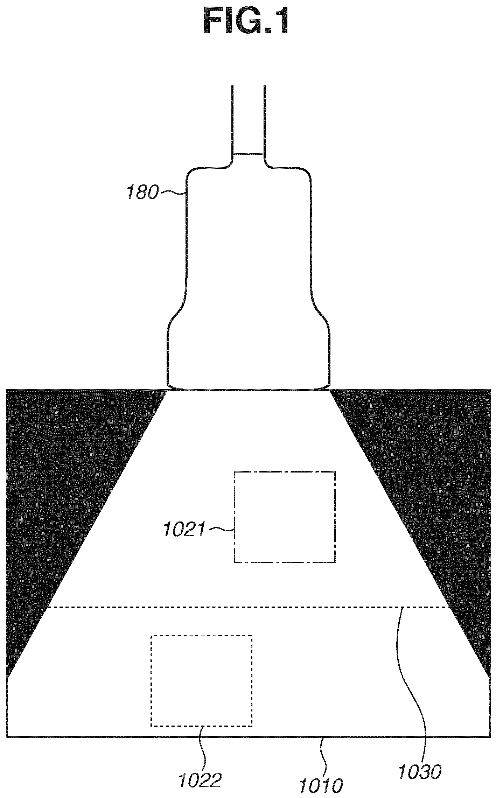

Fig. 1 is a schematic view illustrating a state where an ultrasound image 1010 is generated by using a probe 180 including a transducer capable of transmitting and receiving an ultrasonic wave. The probe 180 illustrated in Fig. 1 includes a light source and a transducer capable of receiving a photoacoustic wave which are configured to generate a photoacoustic image.

The following considers a case where a user confirms the ultrasound image 1010 displayed on a display, and diagnoses the region of interest which is suspected of tumor. Further, the following considers a case where, after confirming the region of interest in the ultrasound image 1010, the user desires a photoacoustic image corresponding to the region of interest. If the entire range where the ultrasound image 1010 is displayed is imaged under the same light irradiation condition, a suitable photoacoustic image may not be displayed at a certain position. More specifically, when a photoacoustic image is superimposed on the ultrasound image 1010 on the entire range where the ultrasound image 1010 is displayed, a moving image suitable for each position may not be displayed. The following considers a case where the repetition frequency of the irradiation light is determined in synchronization with the refresh frequency (for example, a case where the refresh frequency coincides with the repetition frequency of the irradiation light).

For example, when increasing the repetition frequency of the irradiation light by giving priority to the refresh frequency of image display, it is necessary to reduce the light quantity of the irradiation light taking into consideration the heat generation from the light source and the Maximum Permissible Exposure (MPE). In this case, at positions where the distance from the probe 180 (distance from the light irradiation position) is short, a moving image with a sufficient image quality can be displayed at a high refresh frequency. On the other hand, at positions where the distance from the probe 180 is long, an image is updated at a high refresh frequency and a moving image may provide low diagnostic capability. This is because of an insufficient light quantity of the irradiation light and hence a low image quality of each frame. The light quantity of the irradiation light (hereinafter also referred to as an irradiation light quantity) is defined as the total amount of light energy per pulse with a unit of Joule (J). Therefore, the average power with a unit of watt (W) of the irradiation light is represented by the irradiation light quantity multiplied by the number of light emissions per second. One-pulse light includes light with the light intensity varying with time in square wave form, light varying in triangle wave form, light varying in sine wave form, and light varying in the form of all other waves. Referring to Fig. 1, the distance from the probe 180 is defined as the distance from the surface of the probe 180 contacting the imaging target object to each position in a lower direction on the paper.

Typically, a larger irradiation light quantity can generate a larger acoustic wave, improving the signal-to-noise (S/N) ratio of a received signal of the photoacoustic wave. As a result, photoacoustic image data with high display image quality can be obtained. Then, when the irradiation light quantity is increased by giving priority to the image quality at far positions from the probe 180, it is necessary to reduce the repetition frequency of the irradiation light taking into consideration the heat generation from the light source and the MPE. When the region of interest is not at a far position from the probe 180, the refresh frequency at near positions from the probe 180 becomes unnecessarily low, possibly resulting in degradation of diagnostic capability.

Accordingly, the inventor(s) of the present invention has/have found setting a region of interest subjected to photoacoustic image display when the ultrasound image is displayed, and setting the light irradiation condition based on information indicating the region of interest. Then, the inventor(s) has/have found, by performing light irradiation under the light irradiation condition set in such a manner, selectively displaying the photoacoustic image of the region corresponding to the region of interest. The information indicating the region of interest may be the information representing the region of interest as a function, information representing the coordinates of the region of interest, and other information represented in any way as long as a region of interest can be defined.

More specifically, when the user specifies a region of interest in the displayed ultrasound image and the specified region of interest is close to the probe 180 (the region of interest is set close to the light irradiation position on the subject), the irradiation light quantity is decreased and the repetition frequency is increased. On the other hand, when the specified region of interest is far from the probe 180, the irradiation light quantity is increased and the repetition frequency is decreased.

For example, bordering on the dotted line 1030 illustrated in Fig. 1, when the region of interest is set closer than the dotted line 1030, the irradiation light quantity is decreased and the repetition frequency is increased. When the region of interest is set farther than the dotted line 1030, the irradiation light quantity is increased and the repetition frequency is decreased. For example, a region of interest 1021 specified as illustrated in Fig. 1 is determined to have been set close to the probe 180. On the other hand, a region of interest 1022 is determined to have been set far from the probe 180. When the boundary is included in the region of interest, the region of interest may be determined to be positioned at a far position. It is also possible to determine whether the region of interest is positioned at a far position with reference to the center of the region of interest. More specifically, it is also possible to determine whether the region of interest is positioned at a far position according to the positional relationship between the center of the region of interest and the boundary. It is also possible to determine whether the region of interest is positioned at a far position with reference to the position of the region of interest farthest from the probe 180. More specifically, it is also possible to determine whether the region of interest is positioned at a far position according to the positional relationship between the position of the region of interest farthest from the probe 180 and the boundary.

In an assumable mode, a photoacoustic image is superimposed on the entire range of an ultrasound image at a predetermined refresh frequency, and, after performing the superposition, the user adjusts the refresh frequency of the photoacoustic image according to the region of interest. However, superimposing a photoacoustic image on an ultrasound image makes it hard to recognize the region of interest captured in the ultrasound image. On the other hand, when the user is adjusting the refresh frequency, the display region may be changed from the region displayed before the superposition of the photoacoustic image by the motion of the subject or the shake of the probe 180. If the photoacoustic image is superimposed on the entire range of the ultrasound image, it will be hard to recognize a change of the display region.

For this reason, in a mode in which the refresh frequency is adjusted after superimposing the photoacoustic image on the entire range of the ultrasound image, it is difficult to suitably adjust the refresh frequency at the position of the region of interest. In other words, in this method, it is difficult to suitably set the light irradiation condition such as the repetition frequency and the irradiation light quantity of the irradiation light corresponding to the refresh frequency.

If the photoacoustic image is displayed on the entire range in response to a need for performing basic diagnosis with the ultrasound image like before and locally confirming the photoacoustic image for the region of interest, redundant information will be additionally displayed, possibly degrading diagnostic capability.

Embodiments of the present invention will be described below with reference to the accompanying drawings. However, sizes, materials, shapes, and relative arrangements of elements described below are not limited thereto, and should be modified as required depending on the configuration of an apparatus according to the present invention and other various conditions. The scope of the present invention is not limited to the following descriptions.

### (Apparatus Configuration)

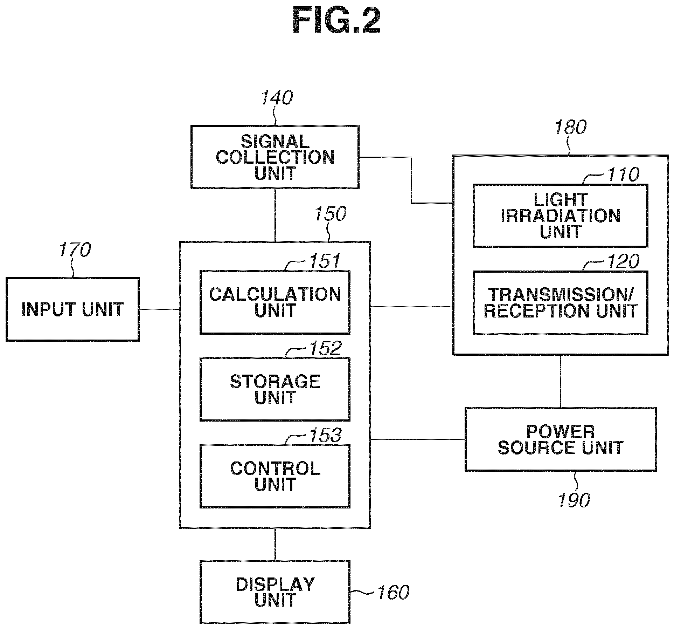

The configuration of the photoacoustic apparatus according to the present embodiment will be described below with reference to Fig. 2. Fig. 2 is a block diagram schematically illustrating the entire configuration of the photoacoustic apparatus. The photoacoustic apparatus according to the present embodiment includes the probe 180 (a light irradiation unit 110 and a transmission/reception unit 120), a signal collection unit 140, a computer 150, a display unit 160, an input unit 170, and a power source unit 190.

When the light irradiation unit 110 irradiates a subject with light, an acoustic wave is generated from the subject. An acoustic wave generated by the photoacoustic effect resulting from light is also referred to as a photoacoustic wave. The power source unit 190 supplies power for driving the light source of the light irradiation unit 110. The transmission/reception unit 120 receives a photoacoustic wave and outputs an electrical signal (photoacoustic signal) as an analog signal.

The signal collection unit 140 converts the analog signal output from the transmission/reception unit 120 into a digital signal, and outputs the digital signal to the computer 150. The computer 150 stores the digital signal output from the signal collection unit 140 as signal data originated from the photoacoustic wave.

The computer 150 performs processing (described below) on the stored digital signal to generate image data. Upon completion of image processing for display on the obtained image data, the computer 150 outputs the image data to the display unit 160. The display unit 160 displays a photoacoustic image. A doctor or an engineer as a user can perform diagnosis by confirming the photoacoustic image displayed on the display unit 160. Based on a storage instruction from the user or the computer 150, the displayed image is stored in a memory in the computer 150 or a data management system connected with a modality via a network.

As a reconstruction algorithm for converting signal data into three-dimensional volume data, a back projection method in the time domain, a back projection method in the Fourier domain, a model base method (repetitive calculation method), and any other methods can be employed. Examples of back projection methods in the time domain include Universal back-projection (UBP), Filtered back-projection (FBP), and Delay-and-Sum.

The computer 150 also controls the drive of components included in the photoacoustic apparatus. The display unit 160 may display graphical user interfaces (GUIs) in addition to images generated by the computer 150. The input unit 170 is configured to allow the user to input information. The user can perform operations for issuing instructions for starting and ending measurement and an instruction for storing a generated image by using the input unit 170.

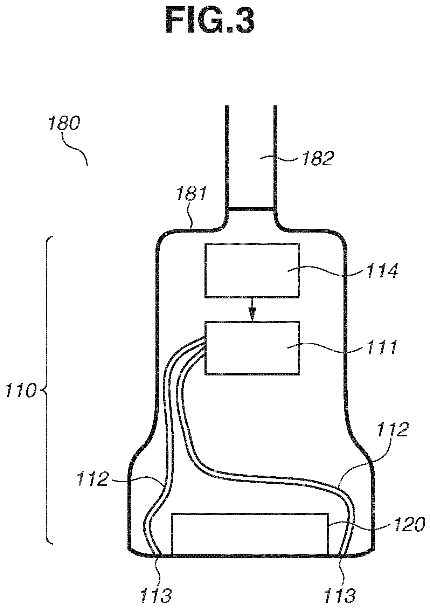

Fig. 3 is a schematic view illustrating the handheld probe 180 according to the present embodiment. The probe 180 includes the light irradiation unit 110, the transmission/reception unit 120, and a housing 181. The housing 181 is a case for storing the light irradiation unit 110 and the transmission/reception unit 120. The user can use the probe 180 as a handheld probe by holding the housing 181. The light irradiation unit 110 includes a light source 111, an optical system 112 for transmitting light generated from the light source 111, and a driver circuit 114 for driving the light source 111. Light is emitted from an emission end 113 of the optical system 112 of the light irradiation unit 110. In the optical system 112 illustrated in Fig. 3, the probe 180 for generating light from the light source 111 such as a light emitting diode (LED) and laser diode (LD) is connected with the signal collection unit 140, the computer 150, and the power source unit 190 via a cable 182. The cable 182 includes wiring for supplying power from the power source unit 190 to the driver circuit 114. The cable 182 also includes wiring for transmitting control signals for controlling the irradiation light quantity and the light emission timing from the control unit 153 to the driver circuit 114. The cable 182 includes wiring for outputting an analog signal as an output signal of the transmission/reception unit 120 to the signal collection unit 140. The cable 182 may be provided with a connector to allow the probe 180 to be disconnected from the photoacoustic apparatus and other components. According to the present embodiment, the combined configuration of the driver circuit 114 and the power source unit 190 is equivalent to a supply unit for supplying power to the light source 111. In other words, the supply unit according to the present embodiment includes the driver circuit 114 and the power source unit 190.

The probe 180 according to the present embodiment may be a wireless handheld probe 180 free from the cable 182. In this case, the power source unit 190 may be included in the probe 180, and various signals may be wirelessly transmitted and received between the probe 180 and other components. However, if the power source unit 190 is included in the probe 180, the quantity of heat generation in the housing 181 is increased by the heat generated by the power consumption in the power source unit 190. Therefore, to restrain a temperature rise in the housing 181, the power source unit 190 may be disposed outside the housing 181. Further, a part of components of the driver circuit 114 providing a large power consumption and a large quantity of heat generation may be disposed outside the housing 181.

Each component of the photoacoustic apparatus according to the present embodiment will be described in detail below.

### (Light Irradiation Unit 110)

The light irradiation unit 110 includes the light source 111, the optical system 112, and the driver circuit 114.

The light source 111 may include at least one of an LD and an LED. The light source 111 may be a light source with a variable wavelength.

The pulse width of light emitted by the light source 111 may range from 1 ns or more to 1000 ns or less. The wavelength of light may range from about 400 to 1600 nm. When imaging the blood vessel with a high resolution, wavelengths largely absorbed by the blood vessel (400 nm or more to 700 nm or less) may be used. When imaging the deep part of a living body, light with wavelengths typically little absorbed by the background tissue (such as water and fat) of the living body (700 to 1100 nm) may be used. One-pulse light includes light with the light intensity varying with time in square wave form, light varying in triangle wave form, light varying in sine wave form, and light varying in the form of all other waves.

The light source 111 may be an LD or an LED that can emit light following a saw-tooth drive waveform (drive current) with a frequency of 1 MHz or higher.

The optical system 112 may be an optical element such as a lens, mirror, and optical fiber. When the subject is the breast, the optical emitting portion of the optical system 112 may include a diffusing plate for diffusing light to irradiate the subject with pulsed light having an expanded beam diameter. On the other hand, in a photoacoustic microscope, the emission end 113 of the optical system 112 may include a lens to irradiate the subject with a focused beam to improve the resolution. The light irradiation unit 110 not including the optical system 112 may irradiate the subject with light directly coming from the light source 111.

The driver circuit 114 is a circuit for generating a drive current for driving the light source 111 by using power from the power source unit 190.

### (Transmission/Reception Unit 120)

The transmission/reception unit 120 includes a transducer for receiving an acoustic wave and outputting an electrical signal, and a supporting member for supporting the transducer. The transmission/reception unit 120 can also transmit an acoustic wave.

The transducer may be made of such a material as a piezoelectric ceramic material represented by titanic acid lead zirconate (PZT) and a piezoelectric polymer film material represented by polyvinylidene fluoride (PVDF). Elements other than piezoelectric elements may be used. For example, a Capacitive Micro-machined Ultrasonic Transducer (CMUT) or a transducer using a Fabry-Perot interferometer can be used. Transducers of any other types can be used as long as an electrical signal can be output by receiving an acoustic wave. The signal acquired by the transducer is a time-resolved signal. More specifically, the amplitude of the signal acquired by the transducer represents a value based on the sound pressure (for example, a value proportional to the sound pressure) received by the transducer at each time.

Typically, a frequency component of the photoacoustic wave ranges from 100 kHz to 100 MHz. A transducer capable of detecting these frequencies can be used.

As the supporting member, a plurality of transducers may be arranged in a flat or curved plane which is called a 1D array, 1.5D array, 1.75D array, or 2D array.

The transmission/reception unit 120 may include an amplifier for amplifying a time series analog signal output from the transducer. The transmission/reception unit 120 may also include an analog-to-digital (A/D) converter for converting the time series analog signal output from the transducer into a digital signal. More specifically, the transmission/reception unit 120 may include the signal collection unit 140 (described below).

To enable detecting an acoustic wave at various angles, ideally, transducers may be arranged such that the subject is surrounded from all directions of the circumference. However, if transducers cannot be arranged such that the subject is surrounded from all directions of the circumference, transducers may be arranged on a hemispherical supporting member to approximately surround the subject from all directions of the circumference. Arrangements and the number of transducers and the shape of the supporting member may be optimized according to the subject. Any type of the transmission/reception unit 120 can be employed according to the present invention.

The space between the transmission/reception unit 120 and the subject may be filled with a medium in which a photoacoustic wave can be propagated. Materials that can be employed as this medium need to satisfy the following conditions: an acoustic wave can be propagated therein; acoustic characteristics are matched at the interface between the subject and the transducer, and the photoacoustic wave transmissivity is as high as possible. For example, water and ultrasonic gel can be employed as the medium.

A transducer for transmitting ultrasonic waves and a transducer for receiving acoustic waves may be separately prepared. The transducer for transmitting ultrasonic waves and the transducer for receiving acoustic waves may be the same transducer. A transducer for transmitting and receiving ultrasonic waves and a transducer for receiving photoacoustic waves may be separately prepared. The transducer for transmitting and receiving ultrasonic waves and the transducer for receiving photoacoustic waves may be the same transducer.

### (Signal Collection Unit 140)

The signal collection unit 140 includes an amplifier for amplifying the electrical signal (analog signal) output from the transmission/reception unit 120, and an A/D converter for converting the analog signal output from amplifier into a digital signal. The signal collection unit 140 may include a Field Programmable Gate Array (FPGA) chip. The digital signal output from the signal collection unit 140 is stored in a storage unit 152 in the computer 150. The signal collection unit 140 is also referred to as a Data Acquisition System (DAS). In the present specification, electrical signals conceptually include both analog and digital signals. The signal collection unit 140 is connected with a light detection sensor attached to the light emitting portion of the light irradiation unit 110, and may start processing in synchronization with the light emission from the light irradiation unit 110 as a trigger. The signal collection unit 140 may also start the processing in synchronization with the issuance of an instruction by using a freezing button as a trigger.

The probe 180 may include the signal collection unit 140 including an amplifier and an analog-to-digital converter (ADC). In other words, the signal collection unit 140 may be disposed in the housing 181. This configuration makes it possible to digitally exchange information between the handheld probe 180 and the computer 150, thus improving noise resistance. In comparison with a case where analog signals are transmitted, using high-speed digital signals enables reducing the number of wires and improving operability of the handheld probe 180.

### (Computer 150)

The computer 150 as an information processing unit includes a calculation unit 151, a storage unit 152, and a control unit 153. Functions of these components will be described below in the description of processing in flowcharts.

Units in charge of calculation functions as the calculation unit 151 include processors such as a central processing unit (CPU) and a Graphics Processing Unit (GPU), and a calculation circuit such as a Field Programmable Gate Array (FPGA) chip. These units may include not only a single processor and calculation circuit but also a plurality of processors and calculation circuits. The calculation unit 151 may receive various parameters such as the subject sonic velocity and holding unit configuration from the input unit 170 and perform processing a received signal.

The storage unit 152 may include a read only memory (ROM) and a non-transitory storage medium such as a magnetic disk and a flash memory. The storage unit 152 may be a volatile medium such as a random access memory (RAM). A storage medium in which programs are stored is a non-transitory storage medium. The storage unit 152 includes not only one storage medium but also a plurality of storage media.

The storage unit 152 can store photoacoustic image data generated by the calculation unit 151. The storage unit 152 can also store displayed images based on the photoacoustic image data.

The control unit 153 includes a calculation element such as a CPU. The control unit 153 controls the operation of each component of the photoacoustic apparatus. The control unit 153 may control each component of the photoacoustic apparatus in response to instruction signals, for example, via an operation to start measurement from the input unit 170. The control unit 153 reads a program code stored in the storage unit 152 and controls the operation of each component of the photoacoustic apparatus.

The computer 150 may be a specially designed workstation. Components of the computer 150 may include different hardware configurations. At least a part of components of the computer 150 may include a single hardware configuration.

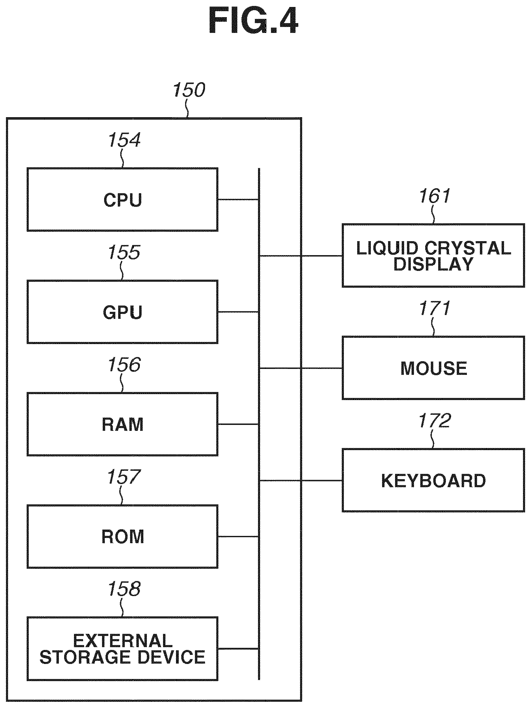

Fig. 4 illustrates an example of a configuration of the computer 150 according to the present embodiment. The computer 150 according to the present embodiment includes a CPU 154, GPU 155, RAM 156, ROM 157, and an external storage device 158. A liquid crystal display 161 as the display unit 160, and a mouse 171 and a keyboard 172 as the input unit 170 are connected to the computer 150.

The computer 150 and the transmission/reception unit 120 may be stored in a common housing. A computer stored in the housing may perform a part of signal processing, and a computer provided outside the housing may perform the remaining signal processing. In this case, the computers provided inside and outside the housing are collectively referred to as a computer according to the present embodiment. More specifically, the hardware configuring the computer does not need to be stored in one housing.

### (Display Unit 160)

The display unit 160 is a liquid crystal display or an organic electro luminescence (EL) display. The display unit 160 is an apparatus for displaying images based on the subject information and numerical values of a specific position acquired from the computer 150. The display unit 160 may display a GUI for operating an image and the apparatus. Before displaying the subject information, the display unit 160 or the computer 150 can perform image processing (such as adjustment of luminance values) on the subject information.

### (Input Unit 170)

As the input unit 170, an operation console including a user-operable mouse and keyboard can be employed. The display unit 160 may include a touch panel and may be used as the input unit 170.

Components of the photoacoustic apparatus may be configured as separate apparatuses or integrally configured as one apparatus. Further, at least a part of components of the photoacoustic apparatus may be integrally configured as one apparatus.

### (Power Source Unit 190)

The power source unit 190 is a power source for generating power. The power source unit 190 supplies power to the driver circuit 114 of the light irradiation unit 110. The power supplied from the power source unit 190 is consumed by the driver circuit 114 and the light source 111, accompanying light emission and heat generation. A direct current (DC) power source can be used as the power source unit 190. The power source unit 190 may be configured as a primary battery, a rechargeable battery, or any other type of battery. If the power source unit 190 is configured as a battery, the power source unit 190 can be stored in the probe 180 in a space-saving way. The driver circuit 114 and the power source unit 190 may be controlled by the control unit 153 in the computer 150. The probe 180 may include a control unit for controlling the power source unit 190 and the driver circuit 114.

### (Subject)

The subject does not configure the photoacoustic apparatus but will will be described below. The photoacoustic apparatus according to the present embodiment can be used for the purpose of diagnosis of malignant tumors and blood vessel diseases of human and animals, and follow-up observations of chemical treatments. Therefore, as the subject, a target portion of diagnosis of a living body, more specifically, the breast, each internal organ, blood vessel networks, head, cervix, abdomen, and limbs (including fingers and toes) of human and animal bodies, is assumed. For example, when a human body is a measurement target, the target optical absorber may be oxyhemoglobin, deoxyhemoglobin, a blood vessel containing large amounts of these substances, or a new blood vessel formed near a tumor. The target optical absorber may be plaque of the carotid wall. The optical absorber may be a pigment such as methylene blue (MB) and indocyanine green (ICG), gold fine particles, or a material introduced from outside and formed by accumulating or chemically modifying these substances. The observation target may be a puncture needle or an optical absorber applied to a puncture needle.

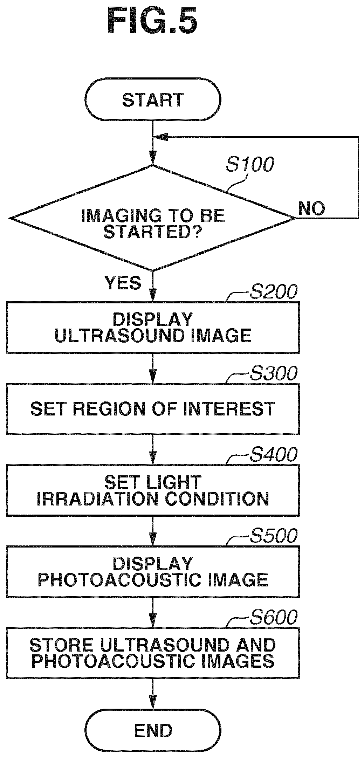

A method for controlling an apparatus for implementing an information processing method including an image display method according to the present embodiment will be described below with reference to the flowchart illustrated in Fig. 5.

### (S100: Process of Determining Whether Instruction for Starting Imaging Is Issued)

The control unit 153 can receive an instruction for starting imaging of an ultrasound image. When the control unit 153 receives an instruction for starting imaging (YES in step S100), the processing proceeds to step S200.

When the user issues an instruction for starting imaging of an ultrasound image via the input unit 170, the control unit 153 receives information indicating an instruction for starting imaging from the input unit 170. For example, when the user presses a switch for starting imaging provided on the probe 180, the control unit 153 receives information indicating an instruction for starting imaging from the input unit 170.

### (S200: Process of Displaying Ultrasound Image)

Upon reception of information indicating an instruction for starting imaging, the control unit 153 performs the following device control.

The transmission/reception unit 120 outputs an ultrasonic wave signal through the transmission and reception of ultrasonic waves to/from the subject. The signal collection unit 140 performs AD conversion processing on the ultrasonic wave signal, and transmits the processed ultrasonic wave signal to the computer 150. The ultrasonic wave signal as a digital signal is stored in the storage unit 152. The calculation unit 151 performs reconstruction processing such as Delay and Sum on the ultrasonic wave signal to generate an ultrasound image. The ultrasonic wave signal stored in the storage unit 152 may be deleted at the timing when an ultrasound image is generated. The control unit 153 as a display control unit transmits the generated ultrasound image to the display unit 160, and controls the display unit 160 to display the ultrasound image. The control unit 153 repetitively performs the above-described processes to update the ultrasound images displayed on the display unit 160, making it possible to display the ultrasound images in moving image form. In this case, the control unit 153 transmits an ultrasonic wave and receives the reflected wave of the transmitted ultrasonic wave. This received signal is referred to as an ultrasonic wave signal.

For example, the control unit 153 transmits the ultrasound image 1010 illustrated in Fig. 1 to the display unit 160 and controls the display unit 160 to display the ultrasound image 1010 in moving image form.

If all of the ultrasound images currently being displayed on the display unit 160 in moving image form are to be stored in the storage unit 152, the amount of storage data will become huge. Therefore, ultrasound images previously displayed may be deleted from the storage unit 152 at the timing when the displayed image is updated.

### (S300: Process of Setting Region of Interest)

The control unit 153 as a region-of-interest setting unit acquires the information indicating the region of interest when the ultrasound image is displayed, and sets the region of interest based on the information. For example, the control unit 153 receives the information indicating the region of interest specified by the user when the ultrasound image is displayed, and sets the region of interest based on the information. More specifically, as illustrated in Fig. 1, the user may specify a region indicating the region of interest in the ultrasound image 1010 by using the input unit 170, and the control unit 153 may acquire the information indicating the region of interest. The user may specify a region with an arbitrary shape, size, and position by using the input unit 170, and the control unit 153 may set the specified region as the region of interest. The user may specify the position of a region with the predetermined shape and size, and the control unit 153 may set as the region of interest the region with the predetermined shape and size at the specified position. The user may specify the size and position, and the control unit 153 may set as the region of interest the region with the predetermined shape having the specified size and position. The user may specify the coordinate values corresponding to the region of interest in the coordinate system of the ultrasound image (coordinate system defined by the position and inclination of the probe 180) by using the input unit 170 to set the region of interest indicated by the specified coordinate values.

The control unit 153 may set the region of interest through image processing on the ultrasound image data generated in step S200. For example, the calculation unit 151 receives information of the region of interest based on a user instruction or inspection order. The calculation unit 151 reads a prestored image pattern corresponding to the region of interest from the storage unit 152, and calculates correlations between this image pattern and a plurality of regions in the ultrasound image data generated in step S200. The calculation unit 151 determines as the region of interest a region where the calculated correlations are higher than a threshold value, and stores the information indicating the region of interest in the storage unit 152.

### (S400: Process of Setting Light Irradiation Condition)

The control unit 153 as an irradiation condition setting unit sets the light irradiation condition including the irradiation light quantity and the repetition frequency of light emitted by the light irradiation unit 110, based on the region of interest set in step S300. For example, the control unit 153 calculates the distance between the set region of interest and the probe 180, and sets the irradiation light quantity and the repetition frequency of light emitted by the light irradiation unit 110, based on the calculated distance. The control unit 153 may set the light irradiation condition so that the irradiation light quantity of the light emitted by the light irradiation unit 110 increases and the repetition frequency thereof decreases with increasing distance.

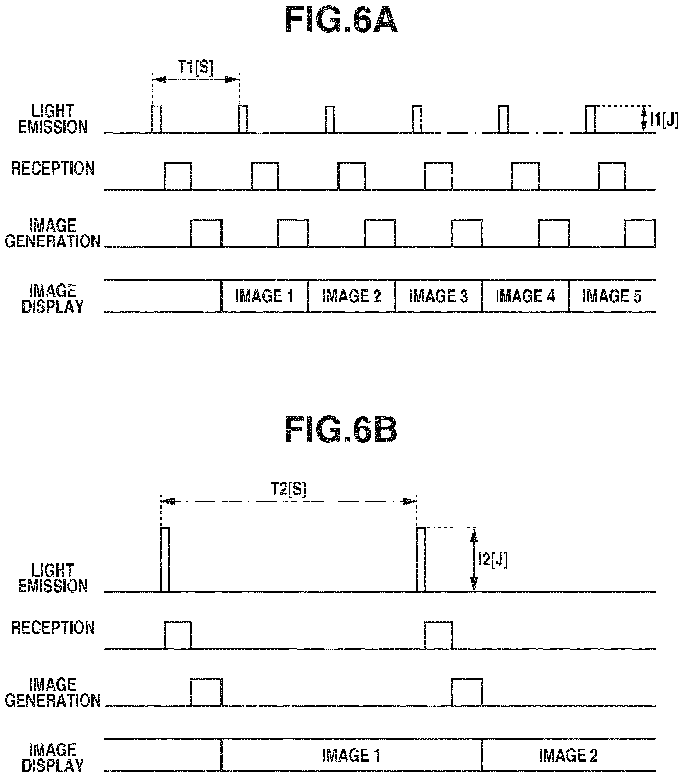

The control unit 153 may determine whether the distance is smaller or larger than a predetermined value, and change the setting values of the irradiation light quantity and the repetition frequency. More specifically, the control unit 153 may set the light irradiation condition corresponding to the set region of interest from a plurality of light irradiation condition patterns. An example of such a method for setting the light irradiation condition will be described below with reference to Figs. 6A and 6B. Figs. 6A and 6B are timing charts illustrating timing of light emission, photoacoustic wave reception, image generation, and image display. In the timing charts illustrated in Figs. 6A and 6B, the refresh frequency of the image display coincides with the repetition frequency of light irradiation.

When the control unit 153 determines that the distance is larger than the predetermined value, the control unit 153 sets the irradiation light quantity to I1 [J] and sets the repetition frequency to 1/T1 [Hz], as indicated by "Light Emission" in Fig. 6A. On the other hand, when the control unit 153 determines that the distance is smaller than the predetermined value, the control unit 153 sets the irradiation light quantity to I2 [J] larger than I1 [J] and sets the repetition frequency to 1/T2 [Hz] lower than 1/T1 [Hz], as indicated by "Light Emission" in Fig. 6B. In this case, a value equivalent to the distance from the probe 180 to the dotted line 1030 illustrated in Fig. 1 is set as the predetermined value.

Although, in this example, the light irradiation condition is set with reference to one predetermined value out of two different light irradiation condition patterns, the light irradiation condition may be selected from three or more different light irradiation condition patterns. In this case, two or more reference values may be set, and the light irradiation condition may be set depending on which numerical range the distance from the probe 180 to the region of interest is included in. The position of the region of interest may be set with reference to the center of the region of interest or with reference to the position of the region of interest farthest from the probe 180.

### (S500: Process of Displaying Photoacoustic Image)

The control unit 153 transmits information (control signals) indicating the light irradiation condition set in step S400 to the light irradiation unit 110. For example, upon reception of a control signal, the driver circuit 114 supplies power to the light source 111 so that the light source 111 performs "Light Emission" illustrated in Figs. 6A and 6B. The light source 111 supplied with power emits light at the timing of "Light Emission" illustrated in Figs. 6A and 6B, light is emitted from the emission end 113 via the optical system 112, and the subject is irradiated with light.

The transmission/reception unit 120 receives a photoacoustic wave generated by light irradiation at the timing of "Reception" illustrated in Figs. 6A and 6B, and outputs a photoacoustic signal. The signal collection unit 140 performs AD conversion processing on the photoacoustic signal, and transmits the processed photoacoustic signal to the computer 150. The photoacoustic signal as a digital signal is stored in the storage unit 152.

The calculation unit 151 as a generation unit performs reconstruction processing such as Universal Back-Projection (UBP) on the photoacoustic signal at the timing of "Image Generation" illustrated in Figs. 6A and 6B to generate photoacoustic image data. An image generation region of the photoacoustic image data may be a region corresponding to the region of interest set in step S300 or a region corresponding to the display region of the ultrasound image. It is not necessary to generate photoacoustic image data corresponding to regions other than the region corresponding to the region of interest. The image generation region may cover the depth in which the region of interest exists (the distance from the probe 180 or the light irradiation position). In other words, the calculation unit 151 may receive the information indicating the region of interest, and determine an image generation region based on the information. The photoacoustic signal stored in the storage unit 152 may be deleted at the timing when the photoacoustic image data is generated. The calculation unit 151 generates the photoacoustic image data of a plurality of frames by using the received signals of the photoacoustic waves generated by a plurality of times of light irradiation.

The control unit 153 as a display control unit transmits the generated photoacoustic image data to the display unit 160, and controls the display unit 160 to display an image based on the photoacoustic image data. During the period indicated by "Image Display" illustrated in Figs. 6A and 6B, the display unit 160 selectively displays the photoacoustic image of the region corresponding to the region of interest set in step S300, based on the generated photoacoustic image data. The control unit 153 displays the moving image of the photoacoustic image corresponding to the region of interest on the display unit 160 by using the photoacoustic image data of a plurality of frames.

When the light irradiation condition illustrated in Fig. 6A is set based on the region of interest in step S400, information processing is executed based on the timing chart illustrated in Fig. 6A also in this process. In the display mode illustrated in Fig. 6A, an image 1 is first displayed for T1 seconds and then an image 2 is displayed for T1 seconds. Likewise, each of images 3, 4, and 5 is sequentially displayed for T1 seconds in this order. The control unit 153 repeats the above-described process to update the image display based on new image data at intervals of T1 seconds.

Meanwhile, when the light irradiation condition illustrated in Fig. 6B is set based on the region of interest in step S400, information processing is executed based on the timing chart illustrated in Fig. 6B also in this process. In the display mode illustrated in Fig. 6B, the image 1 is first displayed for T2 seconds and then the image 2 is displayed for T2 seconds. The above-described process is repeated to update the image display based on new photoacoustic image data at intervals of 2 seconds.

In the display mode illustrated in Fig. 6A, since the refresh frequency is high, the time required for the reconstruction processing needs to be short. Hardware having a simply improved version of the throughput of the computer 150 is also usable. In the display mode illustrated in Fig. 6A, the time required for the reconstruction processing may be decreased by reducing the amount of calculation in the reconstruction. For example, the amount of calculation may be decreased by reducing the amount of data by coarsely setting the pitch of reconstructed voxels, reducing the number of gradations subjected to analog-to-digital conversion, or decreasing the frequency subjected to analog-to-digital conversion.

In the display mode illustrated in Fig. 6A, since the irradiation light quantity is small, light hardly reaches the deep part inside the subject. More specifically, a photoacoustic wave reaching the transmission/reception unit 120 at a late timing from light irradiation out of photoacoustic waves reaching the transmission/reception unit 120 provides low usability. Accordingly, the control unit 153 may reduce the amount of calculation by setting a short analog-to-digital conversion time (photoacoustic wave reception time) and hence reducing the number of data items i.e., the amount of data subjected to analog-to-digital conversion. In this case, it is also possible to reconstruct a smaller region (a region a sufficient light quantity reached) than in the display mode illustrated in Fig. 6B. In this case, both the photoacoustic wave receiving time (equivalent to "Reception" illustrated in Figs. 6A and 6B) and the image generation time (equivalent to "Image Generation" illustrated in Figs. 6A and 6B) can be shortened.

The irradiation light quantity differs between the display modes illustrated in Figs. 6A and 6B. Therefore, the photoacoustic wave generation sound pressure and the magnitude of the received signal change in the two display modes. As a result, the brightness of the displayed image will change. Displaying the reconstructed image in this way disturbs the observation of the reconstructed image by the user.

Since the light quantity setting value is determined according to the display mode, the ratio of the sound pressures of the photoacoustic waves generated in the two display modes is known in advance. Therefore, even if the quantity of light from the light source 111 changes, the computer 150 may correct the light quantity so that the brightness of the displayed image of the image data remains unchanged based on the light quantity setting value. The target of correction may be the received signal, the image data, or the displayed image. The following considers an example case where the irradiation light quantity decreases to one third when the display mode illustrated in Fig. 6B is changed to the display mode illustrated in Fig. 6A. In this case, it is presumed that the received signal decreases to one third. Then, the control unit 153 may triple the degree of amplification for amplifying the analog output (photoacoustic signal) of the transmission/reception unit 120, reduce the voltage range subjected to the conversion by the ADC to one third, or multiply the digital signal converted by the ADC by 3. Through at least one of these processes, processing for leaving the brightness of the displayed image unchanged regardless of the set light quantity value may be performed.

In the display modes illustrated in Figs. 6A and 6B, the computer 150 generates and displays the photoacoustic image data each time one-pulse light irradiation is performed. The computer 150 may generate and display image data by using the received signal of the photoacoustic wave resulting from a plurality of times of pulsed light irradiation. More specifically, the repetition frequency of the light emission by the light source 111 does not need to coincide with the refresh frequency of the image display. The refresh frequency of the image display may be lower than the repetition frequency of the light emission by the light source 111. When generating a one-frame image, the received signal once used to generate other frame images may be used. In this case, a plurality of received signals may be averaged or moving-averaged.

The photoacoustic apparatus according to the present embodiment makes it possible to select a first mode in which the repetition frequency of light irradiation is 1/T1 [Hz] (first repetition frequency) and the irradiation light quantity is I1 [J] (first light quantity). The photoacoustic apparatus according to the present embodiment also makes it possible to select a second mode in which the repetition frequency of light irradiation is 1/T2 [Hz] (second repetition frequency) lower than 1/T1 [Hz] and the irradiation light quantity is I2 [J] (second light quantity) higher than I1 [J]. The photoacoustic apparatus according to the present embodiment makes is possible to switch between the first and the second modes based on the information indicating the region of interest.

The light irradiation unit 110 may have a plurality of light sources 111 and switch the light sources 111 depending on the mode. For example, in the first mode providing a low irradiation light quantity, an LED may be used as the light source 111. On the other hand, in the second mode providing a high irradiation light quantity, an LD may be used as the light source 111. Switching the light sources 111 capable of efficiently generating the irradiation light quantity in each mode enables efficiently using the supplied power, restricting the power consumption of the light sources 111. Switching the light sources 111 in this way enables avoiding local concentration of heat.

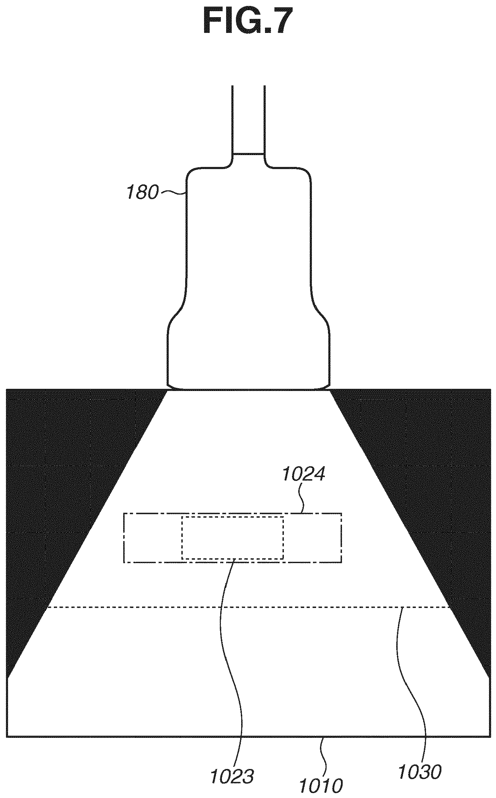

The control unit 153 may change the irradiation range of the irradiation light according to the region of interest. For example, as illustrated in Fig. 7, a case where a region of interest 1023 (dashed lines) and a region of interest 1024 (dashed-dotted lines) are set will be considered. In this case, although both regions of interest have the approximately same distance from the probe 180 (distance from the light irradiation position), the regions of interest differently extend in the lateral direction of paper. In a case where the region of interest 1024 is set, the control unit 153 may irradiate a wider region with light than in the case where the region of interest 1023 is set so that the region of interest 1024 is irradiated with light as uniformly as possible. However, if the light source 111 is supplied with the same power in the cases where the regions of interest 1023 and 1024 are set, the light energy per unit area in the irradiation region will decrease when the region of interest 1024 is set. This may possibly degrade the image quality of the photoacoustic image of the region corresponding to the region of interest 1024. Accordingly, the control unit 153 may control the power supply to the light source 111 to change the quantity of light emitted by the light source 111 based on the information indicating the set irradiation range. For example, when extending the irradiation range by moving the optical system 112, the control unit 153 may control the power supply to the light source 111 such that the power supply to the light source 111 increases and the difference in light emission energy per unit area before and after changing the irradiation range decreases.

The light irradiation unit 110 may expand the light irradiation range in the first mode providing a low irradiation light quantity and reduce the range in the second mode providing a high irradiation light quantity. More specifically, in a mode in which a large irradiation light quantity is required, the light emission energy density to be radiated to the subject may be increased by reducing the irradiation range. As a result, in the second mode providing a high irradiation light quantity, the photoacoustic wave generation sound pressure can be increased, making it possible to improve the image quality of the displayed image.

The probe 180 may be provided with a temperature sensor, and the control unit 153 may instruct a notification unit to notify the user of temperature information of the probe 180 based on the output of the temperature sensor. For example, when the control unit 153 determines that the temperature of the probe 180 (for example, the temperature inside the housing 181) reaches 43 degrees Celsius or higher based on the output of the temperature sensor, the control unit 153 may instruct the notification unit to notify the user of a warning. Also when the control unit 153 determines that the temperature of the probe 180 is lower than 43 degrees Celsius (for example, 41 degrees Celsius), the control unit 153 may instruct the notification unit to notify the user of a warning. In this way, the control unit 153 may instruct the notification unit to notify the user in a plurality of steps in response to the temperature of the probe 180 presumed based on the output of a temperature sensor. For example, as the notification unit, not only a unit for displaying the temperature information of the probe 180 on the display unit 160 but also a unit for notifying the user of the information via an indicator light or sound may be employed. When the control unit 153 determines that the temperature of the probe 180 is higher than a predetermined value based on the output of the temperature sensor, the control unit 153 may control the power source unit 190 and the driver circuit 114 to stop the power supply to the light source 111.

The photoacoustic apparatus according to the present embodiment can perform light irradiation by using information indicating a user instruction as a trigger to generate a photoacoustic image corresponding to the timing of a storage instruction. The light irradiation unit 110 may perform light irradiation at the timing of a user instruction or when a predetermined time period has elapsed since the timing of a user instruction.

It is desirable that the control unit 153 controls each component to perform light irradiation in a time period during which the effect of body motions by breathing and pulsation can be regarded as small to generate a photoacoustic image with respect to the user instruction reception timing. For example, the control unit 153 may control the light irradiation unit 110 to perform light irradiation within 250 ms after reception of a user instruction. The control unit 153 may control the light irradiation unit 110 to perform light irradiation within 100 ms after a user instruction. The time period between reception of a user instruction and light irradiation may be a predetermined value or can be specified by the user via the input unit 170.

The control unit 153 may perform light irradiation not only upon reception of the information indicating a user instruction but also upon reception of information indicating the detection of contact between the probe 180 and the subject. This enables avoiding light irradiation when the probe 180 and the subject are not in contact with each other, making it possible to restrain redundant light irradiation.

In this process, the photoacoustic image corresponding to the region of interest may or may not be superimposed on an ultrasound image. The photoacoustic image may be displayed on the display unit 160 to allow the ultrasound image to be independently observed. For example, the ultrasound and photoacoustic images may be displayed side by side to allow the ultrasound image to be independently observed. In this case, in addition to the display mode in which the photoacoustic image is not superimposed on the ultrasound image, a display mode in which the ultrasound and photoacoustic images are superimposed and displayed in moving image form. The control unit 153 may switch between the display modes based on a switching instruction issued by the user via the input unit 170. For example, the control unit 153 may switch between a parallel display mode in which the photoacoustic image is not superimposed on the ultrasound image and the superimposed display mode.

### (S600: Process of Storing Ultrasound and Photoacoustic Images)

The control unit 153 stores the ultrasound and photoacoustic images. Upon reception of information indicating a storage instruction from the user, the control unit 153 may store the ultrasound and photoacoustic images corresponding to the storage instruction timing in an associative way. The photoacoustic image data and the ultrasound image data may be independently stored without being associated with each other.

When the user observes the ultrasound and photoacoustic images displayed on the display unit 160 in moving image form and confirms the storage target, the user can issue a storage instruction via the input unit 170. In this case, in a state where a still image is displayed on the display unit 160, the user may issue a storage instruction by pressing the freeze button provided on the operation console as the input unit 170. In this case, the control unit 153 receives the information indicating a storage instruction from the input unit 170. The control unit 153 may receive a storage instruction from an external network such as a Hospital Information System (HIS) and a Radiology Information System (RIS).

The storage unit 152 may store the image displayed on the display unit 160 when a storage instruction is received, as an image corresponding to the storage instruction timing. Alternatively, the storage unit 152 may store the image displayed on the display unit 160 when a storage instruction is received and images of temporally neighboring frames, as images corresponding to the storage instruction timing.

Images generated in a time period during which the effect of body motions by breathing and pulsation can be regarded as small for the user instruction reception timing may be stored as images of temporally neighboring frames. For example, the storage unit 152 may store images of frames within ±250 ms after a storage instruction as images of temporally neighboring frames. The storage unit 152 may also store images of frames within ±100 ms after a storage instruction as images of temporally neighboring frames. The storage target can be determined based on the number of frames. For example, the storage unit 152 may store images within ±5 frames after a storage instruction as images of temporally neighboring frames. The storage unit 152 may also store images within ±1 frame after a storage instruction, i.e., adjacent images as images of temporally neighboring frames. The time difference and frame difference between the storage instruction timing and the storage target image acquisition timing may be supplied as predetermined values or specified by the user via the input unit 170. More specifically, the user may specify a "temporally neighboring" range via the input unit 170.

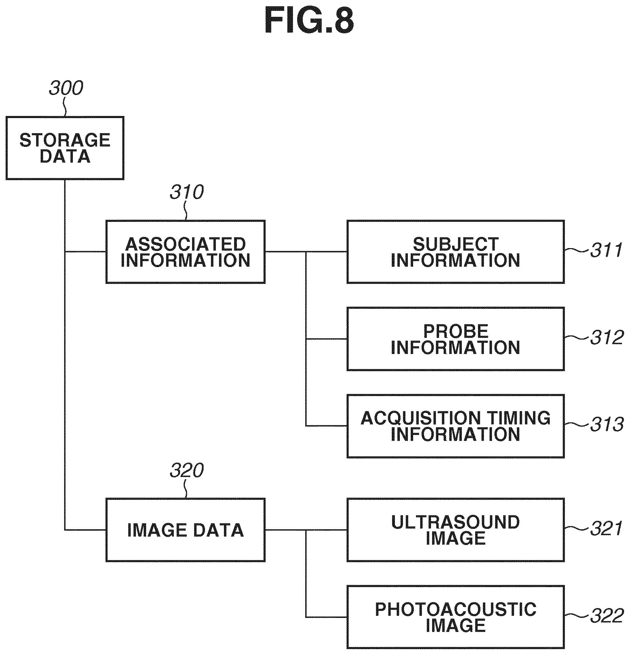

Although the ultrasound and photoacoustic images are stored in an associative way in this process, the associated information may also be stored in an associative way. For example, in step S600, storage data 300 as illustrated in Fig. 8 can be stored in the storage unit 152. The storage data 300 includes associated information 310 and image data 320. The image data 320 includes ultrasound image data 321 and photoacoustic image data 322 associated with each other. The associated information 310 includes subject information 311 as information about the subject and probe information 312 as information about the probe 180. The associated information 310 includes acquisition timing information 313 as information about the acquisition timing (acquisition time) of the ultrasound image data 321 or the photoacoustic image data 322 as storage targets in step S600.

In this case, the subject information 311 includes at least one piece of information of, for example, the subject identifier (ID), subject name, age, blood pressure, cardiac rate, body temperature, height, weight, previous diseases, the number of pregnancy weeks, and inspection target part. The apparatus according to the present embodiment may have an electrocardiogram or pulse oximeter (not illustrated), and an electrocardiogram or information output from the pulse oximeter corresponding to the storage instruction timing may be stored in an associative way as subject information. In addition, all pieces of information related to the subject can be considered as subject information.

The probe information 312 includes information about the probes 180, such as the position and inclination of the probe 180. For example, the probe 180 includes a position sensor such as a magnetic sensor. Information about the output from the position sensor corresponding to the storage instruction timing may be stored as the probe information 312. The probe information 312 may include the information indicating the light irradiation condition set in step S400.

Information about the transmission timing of control signals for ultrasonic wave transmission and reception may be stored as the acquisition timing information 313 of the ultrasound image. Further, information about the transmission timing of control signals for light irradiation may be stored as acquisition timing information of the photoacoustic image. The apparatus according to the present embodiment may include a light detection unit configured to detect a pulsed light emitted from the light irradiation unit 110, and information about the output timing of the signal from the light detection unit may be stored as acquisition timing information of the photoacoustic image.

Although the storage data 300 including one pair of the image data 320 associated with each other has been described above with reference to Fig. 8, a plurality of pairs of the image data 320 may be included in one piece of the storage data 300. In this case, it is desirable to store the associated information related to the plurality of pairs of the image data 320 in the same storage data 300. Alternatively, a plurality of pairs of the image data 320 may be stored as different storage data 300.

As the format of storage data 300, for example, a data format conforming to the Digital Imaging and Communication in Medicine (DICOM) standard can be employed. The format of the storage data 300 according to the present invention is not limited to DICOM and may be any data format.

The control unit 153 can receive an instruction for ending inspection. When the control unit 153 receives an instruction for ending inspection, the control unit 153 ends an inspection. The control unit 153 can receive an instruction from the user and an instruction from an external network such as an HIS and an RIS. The control unit 153 may determine the end of an inspection when a predetermined time has elapsed since the instruction for starting an inspection is received in step S100.

As described above, the information processing method according to the present embodiment enables setting a suitable region of interest when the ultrasound image is displayed. The information processing method also enables setting the light irradiation condition for restricting degradation of the diagnostic capability according to the set region of interest. The photoacoustic image of the region corresponding to the region of interest displayed through light irradiation under the light irradiation condition set in such a manner is a displayed image which contributes to the improvement of the diagnostic capability. The user can perform more proper diagnosis by confirming this photoacoustic image in addition to the ultrasound image. The present embodiment has been described above centering on an example for setting a region of interest in the ultrasound image. However, the present invention is also applicable when setting a region of interest on an image obtained by a modality other than the ultrasonic diagnosis apparatus, such as Computed Tomography (CT) and Magnetic Resonance Imaging (MRI). The present invention is also applicable when setting a region of interest in a photoacoustic image displayed under a predetermined display condition and re-setting the light irradiation condition or display condition.

A second embodiment of the present invention will be described below. The second embodiment is a particularly preferable embodiment in a case where an LD or LED is used as the light source 111 and the S/N ratio of the photoacoustic signal by one-pulse irradiation is not sufficient. In the case of an insufficient light quantity in one-pulse light emission, pulse light emission is performed a plurality of times, acquired photoacoustic signals are addition-averaged to improve the S/N ratio, and a photoacoustic image is generated based on the addition-averaged photoacoustic signal. Simple averaging, moving averaging, or weighted averaging can be employed as addition averaging.

The second embodiment performs pulse light emission a plurality of times to acquire one reconstructed image and then addition-averages the acquired photoacoustic signals. The second embodiment handles the total light quantity of a plurality of pulse light emissions for obtaining one reconstructed image equivalently to the above-described irradiation light quantity. Handling the irradiation light in this way enables applying the above-described light irradiation condition according to the first embodiment. In this case, the repetition frequency of light irradiation does not correspond to the frequency defined from the interval of pulse light emissions for performing addition averaging but corresponds to the frequency (refresh frequency) based on the interval of reconstructed image acquisition.

The processing in steps S400 and S500 in the flowchart illustrated in Fig. 5 according to the second embodiment will be described below centering on differences from the processing according to the first embodiment.

### (S400: Process of Setting Light Irradiation Condition)

Similar to the first embodiment, the control unit 153 as an irradiation condition setting unit sets the light irradiation condition including the irradiation light quantity and the repetition frequency of light emitted by the light irradiation unit 110 based on the region of interest set in step S300. The control unit 153 sets the light irradiation condition to increase the irradiation quantity of light emitted by the light irradiation unit 110 and decrease the repetition frequency, with increasing distance from the probe 180 to the region of interest.

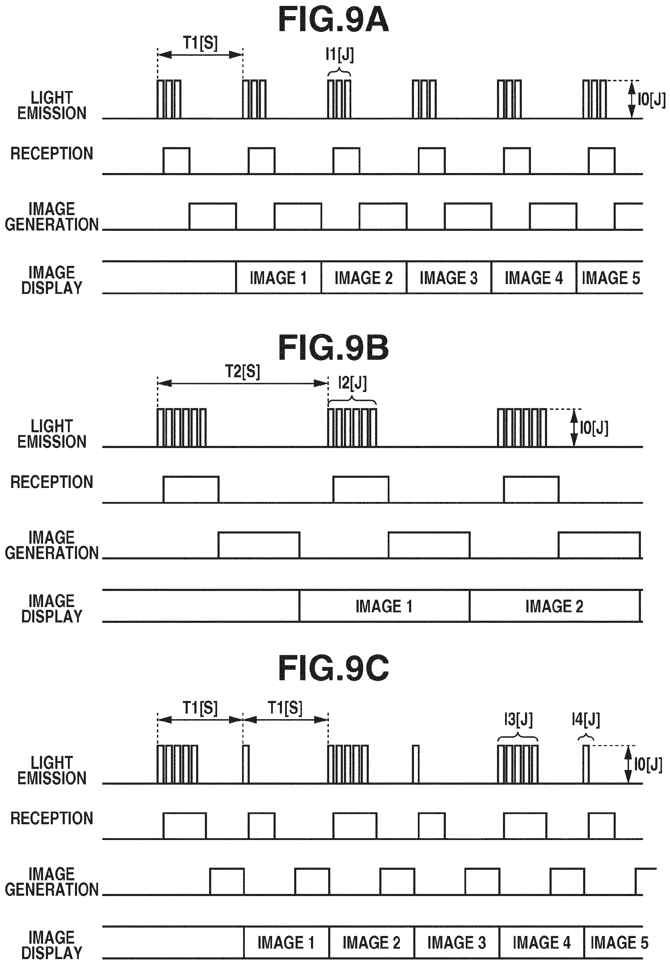

Examples of a method for setting the light irradiation condition according to the second embodiment will be described below with reference to Figs. 9A to 9C. Figs. 9A to 9C are timing charts illustrating the timing of light emission, reception of a photoacoustic wave, image generation, and image display. Referring to Figs. 9A to 9C, description of elements equivalent to those in Figs. 6A and 6B will be omitted. Similar to Figs. 6A and 6B, Figs. 9A to 9C illustrate timing of irradiation light emission, reception of a photoacoustic wave, generation of image data, and display of image data. A vertical line of the timing chart of "Light Emission" indicates the light quantity (10 [J]) of each pulse light emission in a plurality of pulse light emissions. The total light quantities (I1 [J] and I2 [J]) of a plurality of pulse light emissions will also be described below. As described above, according to the second embodiment, the total light quantity of a plurality of pulse light emissions is controlled on the premise that the total light quantity is equivalent to the irradiation light quantity.

The timing charts illustrated in Figs. 9A to 9C differ from the timing charts illustrated in Figs. 6A and 6B in that pulse light emission is performed a plurality of times, acquired photoacoustic signals are addition-averaged, and image reconstruction is performed based on the addition-averaged photoacoustic signal. When performing pulse light emission a plurality of times in this way, controlling the light quantity of each pulse light emission of a plurality of pulse light emissions will increase the circuit complexity. Therefore, according to the second embodiment, the light quantity of each pulse light emission of a plurality of pulse light emissions (10 [J]) is fixed, and the number of pulse light emissions in a plurality of pulse light emissions is controlled to control the light quantity (irradiation light quantities I1 [J] and I2 [J]).

When the control unit 153 determines that the distance to the region of interest is shorter than a predetermined value, the control unit 153 sets the irradiation light quantity to I1 [J] and sets the repetition frequency to 1/T1 [Hz], as indicated by "Light Emission" illustrated in Fig. 9A. For example, the irradiation light amount I1 [J] is achieved when pulsed light having a light quantity of 10 [J] is emitted three times.

On the other hand, when the control unit 153 determines that the distance is longer than the predetermined value, the control unit 153 sets the irradiation light quantity to I2 [J] larger than I1 [J] and sets the repetition frequency to 1/T2 [Hz] lower than 1/T1 [Hz], as indicated by "Light Emission" in Fig. 6B. For example, the irradiation light amount I2 [J] is achieved when pulsed light having a light quantity of 10 [J] is emitted six times.

Based on a light emission timing signal from the control unit 153, the driver circuit drives the light source 111 such as an LD and LED to perform pulse light emission the number of times corresponding to the light quantity setting value. In this case, as the predetermined value, a value equivalent to the distance from the probe 180 to the dotted line 1030 illustrated in Fig. 1 is set.

Referring to Figs. 9A and 9B, the light quantity of each pulse light emission of a plurality of pulse light emissions is fixed (10 [J]), as described above. Referring to Figs. 9A to 9C, the control unit 153 sets the irradiation light quantity to the irradiation light amount I2 [J] (10 [J] x 6) which is twice the value of I1 [J] (10 [J] x 3), allowing the observation of the region of interest of the deeper part. Then, the control unit 153 determines the repetition frequency 1/T2 [Hz] to a half of the repetition frequency 1/T1 [Hz]. Setting the repetition frequencies in this way enables reducing the quantity of heat generation in the housing 181 regardless of change in the light irradiation condition.

### (S500: Process of Displaying Photoacoustic Image)

The transmission/reception unit 120 receives the photoacoustic wave generated by a plurality of pulse light emissions at the timing of "Reception" illustrated in Figs. 9A to 9C, and outputs each photoacoustic signal. The signal collection unit 140 performs AD conversion processing and addition averaging processing on each photoacoustic signal, and transmits the addition-averaged photoacoustic signal to the computer 150. The addition-averaged photoacoustic signal as a digital signal is stored in the storage unit 152.

At the timing of "Image Generation" illustrated in Figs. 9A to 9C, the calculation unit 151 performs reconstruction processing on the addition-averaged photoacoustic signal to generate photoacoustic image data. The calculation unit 151 may receive the information indicating the region of interest and determine an image generation region based on this information.

The control unit 153 as a display control unit transmits the generated photoacoustic image data to the display unit 160 to cause the display unit 160 to display the image based on photoacoustic image data.

When the light irradiation condition illustrated in Fig. 9A is set based on the region of interest in step S400, the control unit 153 performs information processing based on the timing chart illustrated in Fig. 9A also in this process. In the display mode illustrated in Fig. 9A, the image 1 is first displayed for T1 seconds and then the image 2 is displayed for T1 seconds. Likewise, each of the images 3, 4, and 5 is sequentially displayed for T1 seconds in this order. The control unit 153 repeats the above-described processes to update the image display based on new image data at intervals of T1 seconds.

On the other hand, when the light irradiation condition illustrated in Fig. 9B is set based on the region of interest in step S400, the control unit 153 performs information processing based on the timing chart illustrated in Fig. 9B also in this process. In the display mode illustrated in Fig. 9B, the image 1 is first displayed for T2 seconds and then the image 2 is displayed for T2 seconds. The above-described processes are repeated to update the image display based on new photoacoustic image data at intervals of T2 seconds.

In the display mode illustrated in Fig. 9A, since the refresh frequency becomes high as described above, processing for shortening the time required for the reconstruction processing may be performed, as described in the first embodiment.

Although the display modes illustrated in Figs. 9A and 9B differ in the irradiation light quantity (total light quantity of a plurality of pulse light emissions), the light quantity of each pulse light emission of a plurality of pulse light emissions is fixed. Therefore, the light quantity distribution (light quantity intensity) inside the subject accompanying each pulse light emission of a plurality of pulse light emissions remains unchanged. As a result, there is an advantage in that it is unnecessary to perform gain control on the amplifier of the signal collection unit 140 corresponding to each pulse light emission of a plurality of pulse light emissions. Further, since the result of addition averaging differs only in the number of times of addition averaging, the addition-averaged photoacoustic signal is also equivalent. Therefore, it is not necessary to largely change the signal processing between the display modes illustrated in Figs. 9A, and 9B.

The photoacoustic apparatus according to the second embodiment also makes it possible to select the first mode in which the repetition frequency of light irradiation is 1/T1 [Hz] (first repetition frequency) and the irradiation light quantity thereof is I1 [J] (first irradiation light quantity). The photoacoustic apparatus according to the present embodiment also makes it possible to select the second mode in which the repetition frequency of light irradiation is 1/T2 [Hz] (second repetition frequency) lower than 1/T1 [Hz], and the irradiation light quantity thereof is I2 [J] (second irradiation light quantity) higher than I1 [J]. The photoacoustic apparatus according to the present embodiment makes is possible to switch between the first and the second modes based on the information indicating the region of interest.

In light irradiation illustrated in Figs. 9A to 9C, the repetition frequency (frequency based on the interval of reconstructed image acquisition) and the irradiation light quantity (Light quantity of pulse light emissions for obtaining one reconstructed image × Number of light emissions) are examples and may be other values optimized to the system.

When the control unit 153 determines that the distance from the light irradiation position to the region of interest is longer than the predetermined value, the display mode illustrated in Fig. 9C may be used instead of the display mode illustrated in Fig. 9B. In the display mode illustrated in Fig. 9C, the refresh frequency is the same as that in the display mode illustrated in Fig. 9A. More specifically, images can be displayed with higher motion follow-up ability than in the display mode illustrated in Fig. 9B.

In the display mode illustrated in Fig. 9C, the repetition frequency of light irradiation is the same as that in the display mode illustrated in Fig. 9A. To suppress the quantity of heat generation in the housing 181, the control unit 153 changes the irradiation light quantity at intervals of the repetition frequency of light irradiation (intervals of reconstructed image acquisition). More specifically, the control unit 153 repeats light irradiation with an irradiation light amount of I3 [J] (pulse light emission with 10 [J] five times) and light irradiation with an irradiation light amount of I4 [J] (pulse light emission with 10 [J] once) at fixed light irradiation repetition intervals of T1 [s]. In this case, according to the irradiation light quantity, the reconstructed image of a certain frame provides a favorable S/N ratio and the reconstructed image of another frame provides a low S/N ratio. This control enables obtaining a reconstructed image with restricted degradation of the S/N ratio, without lowering the refresh frequency. When acquiring a still image, an image of a frame with a large irradiation light quantity can be selected. More specifically, the degradation of the refresh frequency is restricted while preventing a temperature rise of the probe 180, making it possible to obtain a reconstructed image with restricted degradation of the S/N ratio.

In the embodiment illustrated in Fig. 9C, the light quantity of each pulse light emission of a plurality of pulse light emissions is constant. As described above, the light quantity distribution (light quantity intensity) inside the subject accompanying each pulse light emission of a plurality of pulse light emissions remains unchanged. Therefore, the gain of the amplifier of the signal collection unit 140 in each pulse light emission of a plurality of pulse light emissions can be fixed. Since the photoacoustic signals in a plurality of pulse light emissions are addition-averaged, there is an advantage in that it is possible to perform reconstruction under the same condition regardless of the number of a plurality of pulse light emissions.

Similar to the above-described first embodiment, the present invention is also applicable to a configuration for performing one pulse light emission at intervals of the repetition frequency of light irradiation (intervals of reconstructed image acquisition). In this case, the present embodiment can be achieved by changing the light quantity of pulse light emission, varying the gain of the amplifier of the signal collection unit 140, and correcting changes of the photoacoustic signal by change of the light quantity of pulse light emission.

As described above, according to the second embodiment, the light irradiation condition for restricting the degradation of diagnostic capability can be set according to the set region of interest. The quantity of heat generation in the housing 181 can be reduced regardless of change in the light irradiation condition. The photoacoustic image of the region corresponding to the region of interest displayed through light irradiation under the light irradiation condition set in such a manner is an image which contributes to the improvement of the diagnostic capability. The user may perform more suitable diagnosis by confirming this photoacoustic image in addition to the ultrasound image. Other Embodiments

Embodiment(s) of the present invention can also be realized by a computer of a system or apparatus that reads out and executes computer executable instructions (e.g., one or more programs) recorded on a storage medium (which may also be referred to more fully as a 'non-transitory computer-readable storage medium') to perform the functions of one or more of the above-described embodiment(s) and/or that includes one or more circuits (e.g., application specific integrated circuit (ASIC)) for performing the functions of one or more of the above-described embodiment(s), and by a method performed by the computer of the system or apparatus by, for example, reading out and executing the computer executable instructions from the storage medium to perform the functions of one or more of the above-described embodiment(s) and/or controlling the one or more circuits to perform the functions of one or more of the above-described embodiment(s). The computer may comprise one or more processors (e.g., central processing unit (CPU), micro processing unit (MPU)) and may include a network of separate computers or separate processors to read out and execute the computer executable instructions. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)™), a flash memory device, a memory card, and the like.

While the present invention has been described with reference to embodiments, it is to be understood that the invention is not limited to the disclosed embodiments.

## Claims

1. An information processing method comprising:
displaying an ultrasound image generated through transmission of an ultrasonic wave to a subject and reception of the ultrasonic wave reflected from the subject;
setting a partial region of the ultrasound image as a region of interest when the ultrasound image is being displayed;
setting a light irradiation condition including a light quantity and a repetition frequency of irradiation light to the subject according to the region of interest; and
displaying a photoacoustic image of a region corresponding to the region of interest based on a photoacoustic wave generated through light irradiation to the subject under the light irradiation condition.

2. The information processing method according to claim 1, wherein the photoacoustic image of the region corresponding to the region of interest is superimposed on the ultrasound image.

3. The information processing method according to claim 1 or 2, wherein a region specified in the ultrasound image based on a user instruction is set as the region of interest.

4. The information processing method according to any one of claims 1 to 3, wherein the photoacoustic image of the region corresponding to the region of interest is displayed as a moving image.

5. The information processing method according to claim 4, wherein the photoacoustic image is displayed as a moving image by using the repetition frequency as a refresh frequency of the moving image.

6. The information processing method according to any one of claims 1 to 5, wherein the light irradiation condition is set according to a distance between a position of the light irradiation to the subject and the region of interest.

7. The information processing method according to claim 6, further comprising:
setting a first light quantity and a first repetition frequency as the light irradiation condition when the distance is shorter than a predetermined value; and
setting a second light quantity larger than the first light quantity and a second repetition frequency lower than the first repetition frequency as the light irradiation condition when the distance is longer than the predetermined value.

8. The information processing method according to claim 6, wherein the light irradiation condition is set so that the light quantity increases and the repetition frequency decreases as the distance increases.

9. The information processing method according to any one of claims 1 to 8, wherein, the light irradiation condition, including an irradiation range of the irradiation light to the subject, is set according to the region of interest.

10. A program for causing a computer to execute the information processing method according to any one of claims 1 to 9.

11. A photoacoustic apparatus comprising:
a probe for outputting an ultrasonic wave signal generated through transmission of an ultrasonic wave to a subject and reception of the ultrasonic wave reflected from the subject;
a generation means for generating ultrasound image data by using the ultrasonic wave signal; and
a control means for controlling a display means to display an ultrasound image by using the ultrasound image data,
wherein the control means is arranged to:
generate information indicating a region of interest as a partial region of the ultrasound image when the ultrasound image is displayed; and
generate information indicating a light irradiation condition including a light quantity and a repetition frequency of irradiation light to the subject by using the information indicating the region of interest,
wherein the probe is arranged to:
irradiate the subject with light satisfying the light irradiation condition based on the information indicating the light irradiation condition; and
receive a photoacoustic wave generated through light irradiation to the subject, and outputting a photoacoustic signal,
wherein the generation means is arranged to:
generate photoacoustic image data by using the photoacoustic signal, and
wherein the control means is arranged to:
control the display means to display a photoacoustic image of a region corresponding to the region of interest by using the information indicating the region of interest and the photoacoustic image data.

12. The photoacoustic apparatus according to claim 11,
wherein the probe is further arranged to:
perform light irradiation on the subject a plurality of times, with the repetition frequency based on the information indicating the light irradiation condition; and
receive a photoacoustic wave generated by the plurality of times of light irradiation on the subject, and outputting the photoacoustic signal,
wherein the generation means is arranged to:
generate the photoacoustic image data of a plurality of frames by using the photoacoustic signal, and
wherein the control means is arranged to:
control the display means to display the photoacoustic image of the region corresponding to the region of interest in moving image form by using the photoacoustic image data of the plurality of frames.

13. The photoacoustic apparatus according to claim 11 or 12, wherein the control means is arranged to:
generate information indicating a distance between a position of the light irradiation to the subject and the region of interest by using the information indicating the region of interest; and
set the light irradiation condition by using the information indicating the distance.

14. The photoacoustic apparatus according to claim 13, wherein the control means is arranged to:
compare the distance with a predetermined value by using the information indicating the distance;
set a first light quantity and a first repetition frequency as the light irradiation condition when the distance is shorter than the predetermined value; and
set a second light quantity larger than the first light quantity and a second repetition frequency lower than the first repetition frequency as the light irradiation condition when the distance is longer than the predetermined value.

15. The photoacoustic apparatus according to claim 13, wherein, the control means is arranged to use the information indicating the distance to set the light irradiation condition so that the light quantity increases and the repetition frequency decreases as the distance increases.

16. An information processing apparatus comprising:
means for displaying an ultrasound image generated through transmission of an ultrasonic wave to a subject and reception of the ultrasonic wave reflected from the subject;
means for setting a partial region of the ultrasound image as a region of interest when the ultrasound image is being displayed;
means for setting a light irradiation condition including a light quantity and a repetition frequency of irradiation light to the subject according to the region of interest; and
means for displaying a photoacoustic image of a region corresponding to the region of interest based on a photoacoustic wave generated through light irradiation to the subject under the light irradiation condition.
